# EUROPEAN PATENT APPLICATION

(11) **EP 4 000 510 A1**
(43) Date of publication of application: **25.05.2022**
(21) Application number: 20209474.4
(22) Date of filing: 24.11.2020
(51) Int. Cl.: A61B 5/0536

(54) **APPARATUS, METHODS AND COMPUTER PROGRAMS FOR DETERMINING ELECTRICAL OUTPUT SIGNALS FOR BIOLOGICAL SAMPLES**

(71) Applicant: Nokia Technologies Oy, 02610 Espoo (FI)
(72) Inventor: Zheng, Mingde, Bridgewater, NJ New Jersey 08807 (US)
(74) Representative: Swindell & Pearson Limited

(57) **Abstract**

Examples of the disclosure relate to apparatus, methods and computer programs for analysis of biological samples. The apparatus comprising means for: obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells; using the obtained model to determine expected output signals obtained in response to an electrical signal provided between two or more electrodes positioned on the biological sample; and using the expected output signals to adjust one or more settings of the electrodes.

## Description

### TECHNOLOGICAL FIELD

Examples of the disclosure relate to apparatus, methods and computer programs for determining electrical output signals for biological samples. Some relate to apparatus, methods and computer programs for determining electrical output signals that can be used for electrical impedance tomography of biological samples.

### BACKGROUND

Analysis of biological samples can enable useful information to be obtained from a subject. Improving the accuracy and performance of such analysis and understanding of the information flow mechanism can enable better sensor development and extraction of more useful information.

### BRIEF SUMMARY

According to various, but not necessarily all, examples of the disclosure there may be provided an apparatus comprising means for: obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells; using the obtained model to determine expected output signals obtained in response to an electrical signal provided between two or more electrodes positioned on the biological sample; and using the expected output signals to adjust one or more settings of the electrodes.

Different sub-circuits within the model may represent different types of cellular structures.

Different sub-circuits representing different types of cellular structures may be connected to form a representation of a tissue or organ structure.

The sub-circuits may comprise one or more electrical components configured to have substantially equivalent electrical properties to structures within a given type of cell.

The electrical components may comprise at least one of capacitors, resistors, voltage sources, current sources.

Different types of sub-circuits may represent each of the different types of cells and/or different types of tissues within the biological sample.

The distribution of different types of sub-circuits within the model may correspond to the distribution of different types of cells within the biological sample.

Information relating to quantity and/or distribution of types of cells and tissues within the biological sample may be obtained from prior measurements.

The means may also be for adjusting the model to take into account distribution and quantity of types of cell within a measured sample.

The means may also be for adjusting the model to account for changes within the biological sample.

The model may comprise a three-dimensional model.

The means may also be for using the expected output signal to determine a performance of at least one of the electrodes and using the determined performance to adjust the one or more settings of the electrodes.

The settings that are adjusted may comprise at least one of: positions of one or more of the electrodes, types of electrodes, selection of electrodes that are used.

The electrodes may be configured to provide one or more programmable waveforms to enable tomographic analysis of the biological sample.

The tomographic analysis may comprise electrical impedance tomography.

Outputs from the electrodes may be used to provide a control input to at least one of: a device, a computer program.

The control inputs provide may provide for an interface between the biological sample and the device to enable control of the device by the biological sample.

The biological sample may comprise an in-vivo sample.

According to various, but not necessarily all, examples of the disclosure there may be provided a method comprising: obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells; using the obtained model to determine expected output signals obtained in response to an electrical signal provided between two or more electrodes positioned on the biological sample; and using the expected output signals to adjust one or more settings of the electrodes.

According to various, but not necessarily all, examples of the disclosure there may be provided a computer program comprising computer program instructions that, when executed by processing circuitry, cause: obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells; using the obtained model to determine expected output signals obtained in response to an electrical signal provided between two or more electrodes positioned on the biological sample; and using the expected output signals to adjust one or more settings of the electrodes.

### BRIEF DESCRIPTION

Some examples will now be described with reference to the accompanying drawings in which:
FIG. 1 shows an example method;
FIG. 2 shows an example method;
FIG. 3 shows example models;
FIG. 4 shows example models;
FIGS. 5A to 5H shows example estimated output signals;
FIGS. 6A and 6B shows example output signals;
FIG. 7 shows an example visualization of estimated output signals;
FIGS. 8A and 8B show example results;
FIGS. 9A to 9D show example results; and
FIG. 10 shows an example apparatus.

### DETAILED DESCRIPTION

Examples of the disclosure relate to apparatus, methods and computer programs for determining electrical output signals for biological samples. The output signals can be obtained using a model of the biological sample where the model represents the electrical properties of the biological sample. This model can then be used to determine or visualize expected output signals that could be obtained from a real biological sample. These expected output signals can be used to adjust one or more settings of electrodes that are used to analyse the real biological sample to enable improved analysis and response-prediction of the biological sample.

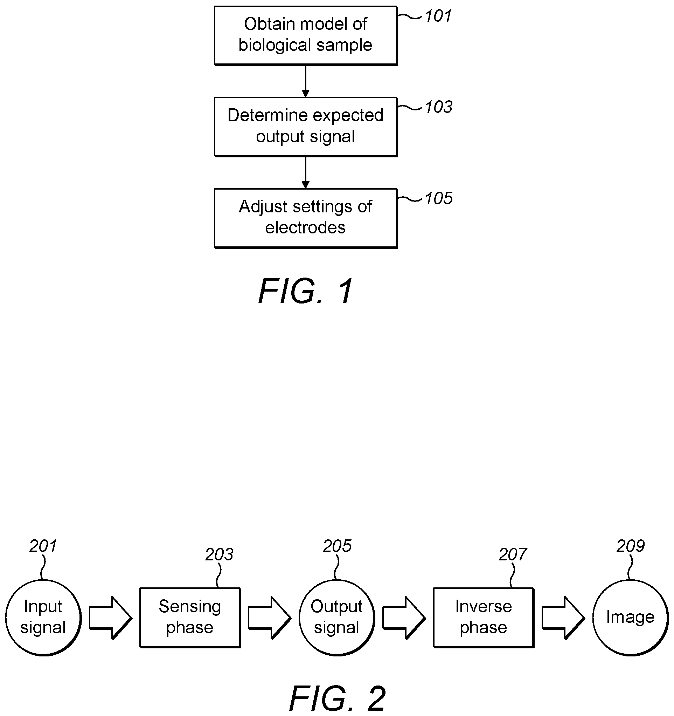

Fig. 1 shows a method according to examples of the disclosure.

The method comprises, at block 101, obtaining a model representing electrical properties of a biological sample. The biological sample that is represented by the model can comprise any biological cellular matter. The biological sample could be an in-vivo sample. For example, the biological sample could comprise part of a subject's body such as a chest cavity or part of a limb or any other suitable part.

In some examples the biological sample could change over time. For instance, where the biological sample comprises a chest cavity this could expand and contract as the user breathes in an out. Where the biological sample comprises muscles these could expand and contract as the subject moves their body or parts of their body.

The biological sample can comprise a plurality of different types of cells. The different types of cells can have different types of cellular structures which provide different electrical responses when stimulated by an electrical input signal. The different types of cells can be comprised within different types of tissues and/or organs.

The model that represents the biological sample can be a virtual computer simulated model. The simulated model can be generated using any suitable programs or other means. In some examples the model could be a real hardware circuit that represents the biological model. The hardware could comprise electrical circuits configured to represent the different types of cells within the biological sample.

The model comprises a plurality of different sub-circuits. The sub-circuits represent individual structures within cells of the biological sample such that the sub-circuits have electrical properties corresponding to the electrical properties of the cells. Where the model is a virtual computer simulated model, the sub-circuits can comprise virtual sub-circuits. Where the model comprises a real hardware circuit, the sub-circuits can comprise hardware components that are arranged to represent the cellular structures.

The different types of sub-circuits within the model represent different types of cellular structures. For example, a first type of sub-circuit can be used to represent a nucleus, a second type of sub-circuit can be used to represent an intracellular medium and a third type of sub-circuit can be used to represent a plasma membrane and other types of sub-circuit can be used to represent other types of cellular structure.

The sub-circuits comprise one or more electrical components configured to have equivalent, or substantially equivalent, electrical properties to structures within a given type of cell. The sub-circuits are designed so that electrical properties such as impedance or dielectric response are equivalent, or substantially equivalent, to the values of the corresponding structures within a real biological sample that is represented by the model. Different types of cells would comprise cellular structures having different electrical properties and this would be represented within the model.

The sub-circuits can comprise one or more of resistors, capacitors, voltage sources, current sources or any other suitable type of components. The respective components can be connected in series or in parallel so as to have equivalent, or substantially equivalent, electrical properties to the cellular structure represented by the sub-circuit.

The individual sub-circuits can be connected to other sub-circuits so as to create a larger circuit representing a plurality of different cellular structures. This creates a model comprising a plurality of different sub-circuits representing different types of cellular structures that are connected to form a representation of a tissue or organ structure.

The quantities and distribution of the different types of sub-circuits within the model can be arranged to correspond to the expected quantities and distribution of different types of cells and tissue within the biological sample. Information relating to quantity and/or distribution of types of cells and tissues within the biological sample can obtained from prior measurements. For example, expected values from general measurements of a population could be used as an estimate for the quantity and distribution of types of cells within a biological sample. As an example the average bone size and density could be selected based on a user's gender, age and height.

In some examples the model can be adjusted to take into account the distribution and quantity of types of cell within a measured sample. For instance, callipers or other means can be used to measure expected levels of fat cells within a biological sample such as a limb of a subject. These measurements could then be used to adjust a standard model to correspond to the measurements of the subject.

In some examples the quantity and distribution of cells within a biological sample could change over time. The changes in the sample could include changes in the position or distributions of types of cells within the biological sample. For example, a muscle within the biological sample could contract or relax or the volume of blood within a tissue could be dependent upon a pulse. These changes could be accounted for within the model.

The model can comprise a three-dimensional model so that the sub-circuits are connected to each other in three different dimensions. The three-dimensional model can be generated by integrating biological parametric inputs, such as anatomical, compositional and bioelectrical information, with boundary conditions to produce a three-dimensional electrical model representing electrical properties of the biological sample. In some examples the three-dimensional model can be computer generated using one or more programming platforms (such as Matlab, python or any other suitable platform) and one or more electrical circuit simulation platforms (such as SPICE, circuitmod or any other suitable platform).

The sub-circuits are therefore arranged within the model to create a compositionally, anatomically, and dimensionally accurate representation of different tissues and/or organs within the biological sample.

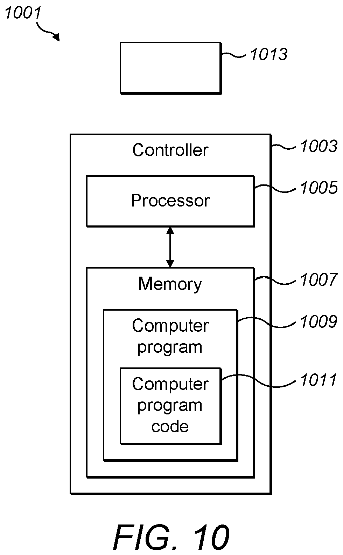

At block 103 the method comprises using the obtained model to determine expected output signals. The electrical output signals would be obtained in response to an electrical signal provided between two or more electrodes positioned on the biological sample. The electrodes can form part of sensing system that enables analysis of the biological sample. The electrodes can be coupled to an apparatus such as the controller apparatus shown in Fig. 10 to form a sensing system. The controller apparatus can control one or more settings of the electrodes.

The electrodes can comprise any means that can be configured to provide one or more electrical input signals to a biological sample and/or sense one or more electrical output signals from the biological sample. The electrodes can comprise input electrodes that can be configured to provide one or more input signals and output electrodes that can sense one or more output signals. In some examples the electrodes could be configured to act as input electrodes and output electrodes at different times.

The electrodes can be coupled to the biological sample so that one or more electrical signals can be provided between the biological sample and the electrodes. In some examples the electrodes can be galvanically connected to the biological sample so as to provide a path for charge transfer between the electrodes and the biological sample.

In some examples the electrodes can be used to obtain output signals for tomographic analysis, or any other suitable type of analysis, of parts of the biological sample. For instance, the electrodes could be configured to analyse part of the biological sample to enable different types of cells, tissues or organs to be identified.

The model can comprise two or more electrodes where the electrodes in the model represent electrodes that are used to analyse a real biological sample. The settings of the electrodes used in the model can be configured to correspond or match the settings of the electrodes that are to be used on a real biological sample. For example, parameters such as the number and arrangement of electrodes can be arranged to be the same in the model as would be used for the real biological sample. The inherent electrical properties, such as impedance, between the electrodes and the sub-circuits within the model can be configured to match the electrical properties between the real electrodes and the real biological sample.

The two or more electrodes tomographic analysis, or any other suitable type of analysis, of the biological sample. The estimated output signal can be used for various other analysis of the biological sample or parts of the samples. For example, it can be used to analyse the bioelectrical constituents of cells or tissues or organs within the model. In some examples the output signals can be used for any other tomographic analysis or any other suitable methods that enable a visualisation and determination of the biological sample to be obtained.

The expected output signals can be obtained by providing input signals to the model and determining the output signals. In some examples the expected output signals can be obtained for a plurality of settings and/or configurations of the electrodes. For example, where the model is a virtual computer simulation a plurality of simulations can be performed for a plurality of different settings of the electrodes. This can enable the different output signals for the different settings to be compared.

At block 105 the method comprises using the expected output signals to adjust one or more settings of the electrodes. The one or more settings of the electrodes can comprise of parameters of a function, input or output signal interrogation, analytical strategy, information processing approach, electrode configuration and any other related adjustable modes of the system. For example, where a plurality of different output signals for a plurality of different electrode settings are obtained, the settings can be selected based on which output signals provide outputs above predetermined thresholds or which settings are considered to provide the best output signals. These settings can then be selected for analysing a real biological sample.

In some examples the performance can be determined by providing a visualization of the operation of the electrodes that can predict the bioelectrical responses of the biological sample to the electrodes input signals. This visualisation can then be used to adjust the settings of the electrodes to enable more accurate outputs to be obtained from the biological sample and/or from the model of the biological sample.

In some examples the model can be configured to obtain an optimal setting for the electrodes. For instance, the model could be used to run simulations for different positions of one or more of the electrodes until the optimal position for analysing a specific part of the biological sample is determined. This optimal position could then be used for positioning electrodes on real biological samples.

In some examples expected output signals obtained from the model can be used to determine a performance of at least one of the electrodes. For example, the expected output signals can be used to determine whether or not a performance exceeds a threshold. The performance could be the resolution, sensitivity, specificity, differentiability or any other suitable factor. If the electrodes do not achieve a required performance threshold then the settings of the electrodes could be adjusted until the threshold performance is obtained.

The determined performance can therefore be used to adjust the one or more settings of the electrodes either within the model or to adjust settings of electrodes used to analyse the real biological sample.

The expected output signals can be used to determine the performance of the electrodes for all or part of the model. For instance, in some examples the expected output signals could be determined for all of the electrodes and for all possible different permutations of electrodes. In other examples the expected output signals could be determined for electrodes located in a particular region of the model or located so as to enable a particular region of the model to be analysed.

The settings that are adjusted can comprise any electrode settings that can affect the output signals of the biological model. In some examples the settings could comprise any one or more of input/output signal functions, analytical programs or positions of one or more of the electrodes, types of electrodes, selection of electrodes that are used or any other suitable setting.

Fig. 2 shows a general overview of electrical impedance tomography of biological samples. Other types of analysis can be used in other examples of the disclosure.

In order to enable electrical impedance tomography, at block 201, a plurality of electrodes are connected to the biological sample to provide an input signal. The biological sample could comprise an in vivo sample. For example, it could comprise a subject's chest cavity or arm or any other suitable part of their body.

The electrodes can comprise any means that can be configured to provide an electrical input signal to the biological sample. In some examples the plurality of electrodes can comprise one or more input electrodes that are configured to provide an input signal to the biological sample and one or more sensing electrodes that are configured to sense an output signal from the biological sample. Whether an electrode is configured as an input electrode or an output electrode will be dependent upon the configuration of a system. It is to be appreciated that an electrode could be configured to provide an input signal at a first time and to sense an output signal at a second, different time.

The type, number and configuration of the electrodes and sensing function that are used will depend on the biological sample that is being analysed, the resolution of the images that is to be provided and any other suitable factor.

The input signals that are delivered by the electrodes can comprise programmable waveforms. The input signals can comprise alternating current signals.

In the sensing phase 203 the input signals pass through the biological sample which results in voltages that can be sensed by one or more of the other electrodes connected to the biological sample at block 205. Different electrodes located in different positions on the biological sample will detect different voltage potentials. By positioning a plurality of different electrodes at different positions on the biological sample, a plurality of different measurements can be obtained for different locations of the biological sample. The different potentials at different locations provide information about the electrical properties of the biological sample between the source electrode and the sensing electrode can be obtained.

In the inverse phase 207 the different measurements from the biological sample are inverted to obtain information about the biological sample. In examples where a plurality of sensing electrodes are used this information can be used, at block 209, to reconstruct one or more images of the biological sample. These images could be three-dimensional images or two-dimensional images.

In examples of the disclosure a model can be used to obtain estimates of expected output signals for a given configuration of electrode function and waveform-delivery layout on the biological sample. These output signals could then be used to adjust the settings of the electrode to enable an improved or more accurate output signal to be obtained. The improved electrode settings can then be used when analysing real biological samples to improve the quality of the information obtained from the sample. For example, it could be used to obtain images with an improved resolution or could be used to monitor changes within a sample with a higher level of sensitivity. Examples of the disclosure therefore enable improvements within the sensing phase which provides more detailed information and higher quality images in the inverse phase of the method.

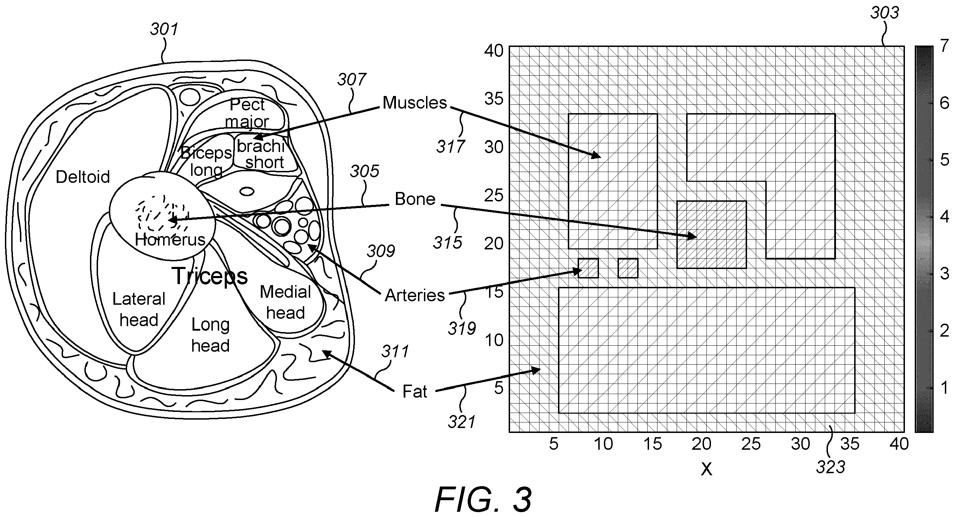

Fig. 3 schematically shows how the anatomical structures and composition of a biological sample 301 can be represented within a model 303.

In this example the biological sample 301 comprises a two-dimensional cross section of an upper arm of a person. Other types of biological sample could be used in other examples of the disclosure.

The biological sample 301 comprises different types of cells and tissues. In this example the biological sample 301 comprises bone 305, muscles 307, arteries 309 and fat 311. Each of these different types of tissues has different electrical properties and so would respond differently to an input electrical signal.

In the example of Fig. 3 the model 303 is a two-dimensional model that represents a cross section of the biological sample 301. The two-dimensional model 303 comprises a plurality of squares where the individual squares represents different cells from the biological sample 301. The position of the squares representing the different types of cells is arranged to correspond to the distribution of the different types of tissues and structures within the real biological sample 301.

Each of the squares within the model 301 comprise a sub-circuit which comprises a combination of resistors, capacitors or other electrical components that are configured in series or parallel so as to provide the same electrical response as the cellular structures that they represent.

A plurality of the sub-circuits can be connected together to provide a representation of a larger structure within the biological sample 301. For example, a plurality of the sub-circuits can be connected together to for a circuit representing the bone or the muscle within the biological sample 301.

Fig. 3 shows different arrangements of squares within the model that are arranged to represent the different structures of the upper arm. A first group of squares 315 represent bone 305, a second group of squares 317 represent muscles 307, a third group of squares 319 represent arteries 309 and a fourth group of squares represent fat 311. The positioning of the groups of squares within the model corresponds to the positioning of the structures within the biological sample 301 that the squares are intended to represent.

Connective space 323 can also be provided between the respective groups of squares. The connective space 323 can comprise corresponding electrical properties to the connective tissues within the biological sample. The enables the shape, sizes, geometry, composition, density and distribution of different types of cells and tissue within the biological sample to be accurately represented by the bioelectrical equivalent map.

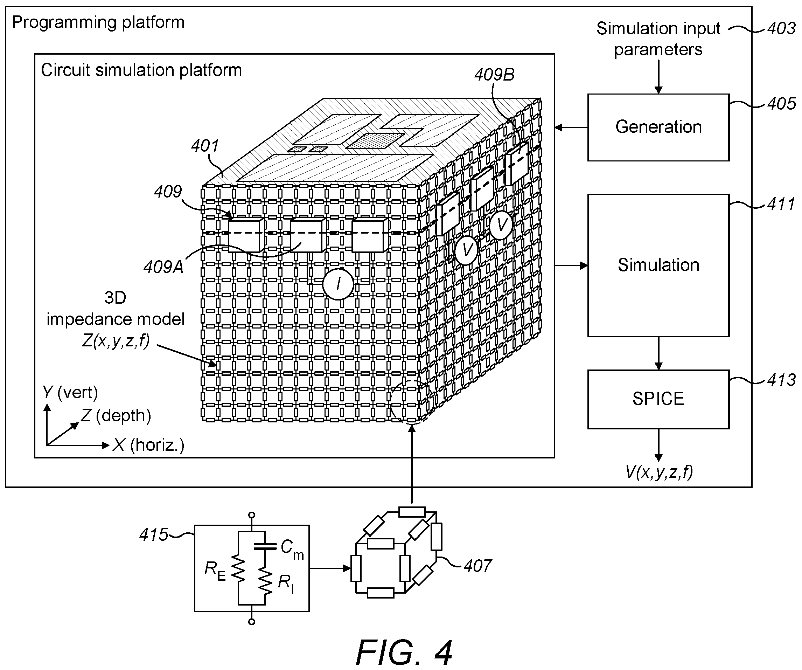

Fig. 4 shows an example of a three-dimensional model 401 of a biological sample that could be obtained using examples of the disclosure. In this example the biological sampled represented by the three-dimensional model 401 is the upper arm of a subject. Different biological samples could be represented by different three-dimensional models 401 in other examples of the disclosure.

In this example the three-dimensional model 401 is virtual simulation that is generated using one or more programming platforms and one or more electrical circuit simulation platforms. The programming platforms used could be MATLAB, python or any other suitable programing platform. The electrical circuit simulation platform could be SPICE, circuitmod or any other suitable electrical circuit simulation platform.

In this example simulation input parameters 403 are provided to the programming platform. The simulation input parameters 403 can comprise parameters indicative of anatomical, compositional and bioelectrical information of the biological sample that is to be represented by the three-dimensional model 401. The simulation input parameters 403 can also comprise boundary conditions for the different structures within the biological sample that is represented by the three-dimensional model 401. The three-dimensional model 401 can be adjusted by adjusting one or more of the simulation input parameters 403.

In some examples the simulation input parameters 403 could comprise a standard set of parameters that correspond to a generic biological sample. For instance, a textbook or other resource could provide information relating to average bone sizes and muscle or fat composition for a typical human subject or other type of subject. In other examples some of these parameters could be measured for a particular subject. For instance measurements of the amount of fat tissue could be made using callipers or other suitable commercially available means and such measurements could then be used to adjust the simulation input parameters to a particular subject.

The simulation input parameters 403 are used to generate a netlist 405 within the programming platform. The netlist defines the electrical properties for each of the sub-circuits within the three-dimensional model 401. The netlist 405 defines the sub-circuits so that the electrical properties of the sub-circuit correspond to the electrical properties of the cellular structure that is represented by the sub-circuit.

The netlist 405 is then used by the electrical circuit simulation platform to generate the three-dimensional model 401. The three-dimensional model 401 comprises a three-dimensional grid comprising a plurality of voxels 407. Each voxel 407 comprises a three-dimensional sub-circuit 415 that represents a cellular structure. The sub-circuit 415 comprises resistors, capacitors, voltage sources, current sources and any other suitable electrical components that are arranged to have electrical properties corresponding to the cellular structure represented by the voxel 407. The sub-circuit can be configured to have time-varying electrical values.

To generate the three-dimensional model 401 a plurality of voxels 407 are interconnected to represent a tissue and a plurality of tissues are interconnected to represent one or more organs within the biological sample.

A plurality of electrodes 409 are provided within the three-dimensional model. The electrodes 409 can be input electrodes 409A that are configured to provide an input signal to the model of the biological sample or output electrodes 409B that are configured to measure the potential in response to an input signal. The electrodes 409 can be configured to act as input electrodes 409A or output electrodes 409B at different times.

The number of electrodes 409 and the relative positions of the electrodes 409 can be configured to correspond to the number and configuration of electrodes that would be used to analyse a real biological sample. Other settings of the electrodes 409, such as the type of the electrode 409, the size of the electrodes 409, the input signals delivered by the electrodes 409 can all correspond to settings that could be used for an analysis of a real biological sample.

Once the three-dimensional model 401 has been generated by the electrical circuit simulation platform a simulation 411 can be ran to obtain estimate output signals for the three-dimensional model 401. The simulation 411 can be run by applying different input signals to the different input electrodes 409A and recording the estimated measured potential at the different output electrodes 409B. The simulations can be ran for some of the possible permutations of electrode settings such as input electrodes 409A and output electrodes 409B. In some examples the simulation can be ran for just a part of the three-dimensional model 401 or in other examples the simulation could be ran for the whole of the three-dimensional model 401.

The obtained estimated output signal can be provided as an output file 413. This information can then be used to adjust settings of a sensor systems such as electrodes 409 either in the three-dimensional model 401 or the electrodes 409 that are used for analysing or monitoring a real biological sample. For example, the output file can provide information about the positions of the electrodes 409 that provide the highest change in potential when a muscle within the biological sample is flexed and relaxed. These positions of the electrodes 409 could then be used to monitor the movement of the muscle in a real biological sample.

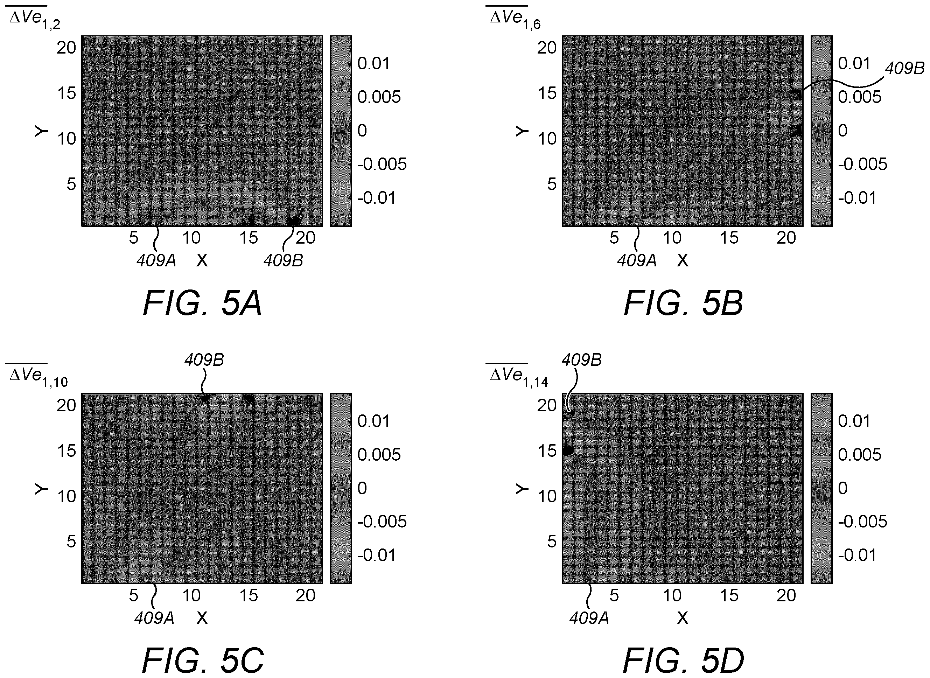

Figs. 5A to 5H show a plurality of example estimated output signals that are obtained using models of biological samples according to examples of the disclosure. The different estimated output signals can be obtained for different configurations of the electrodes 409. The different output signals could be obtained for a three-dimensional model 401 such as the model shown in Fig. 4 or for any other suitable type of model.

In the examples of Figs. 5A to 5H the electrodes 409 within the sensor system are configured for electrical impedance tomography. Other types of analysis could be used in other examples of the disclosure.

In Figs. 5A to 5H a plurality of pairs of electrodes are positioned around the biological sample. The sensing system can be controlled to sequentially change which electrode pairs are used as input electrodes 409A and which electrode pairs are used as output electrodes 409B. In this example there is no movement of the electrode pairs, however the function that is performed by the electrode pairs changes under the control of the sensing system. In this example the sensing system therefore controls settings of the electrodes.

In Fig. 5A the input electrodes 409A and the output electrodes 409B are positioned on the same edge of the model. Both the input electrodes 409A and the output electrodes 409B are positioned on the lower edge of the model. The input electrodes 409A are positioned at the left-hand side of the lower edge of the model and the output electrodes 409B are positioned at the right-hand side of the lower edge of the model.

In Fig. 5B the input electrodes 409A and the output electrodes 409B are positioned on different edges of the model. The input electrodes 409A are positioned on the lower edge of the model and the output electrodes 409B are positioned on the right-hand side edge of the model.

In Fig. 5C the input electrodes 409A and the output electrodes 409B are also positioned on different edges of the model. The input electrodes 409A are positioned on the lower edge of the model and the output electrodes 409B are positioned on the upper edge of the model. The upper edge of the model is opposite to the lower edge of the model.

In Fig. 5D the input electrodes 409A and the output electrodes 409B are also positioned on different edges of the model. The input electrodes 409A are positioned on the lower edge of the model and the output electrodes 409B are positioned on the left-hand side edge of the model.

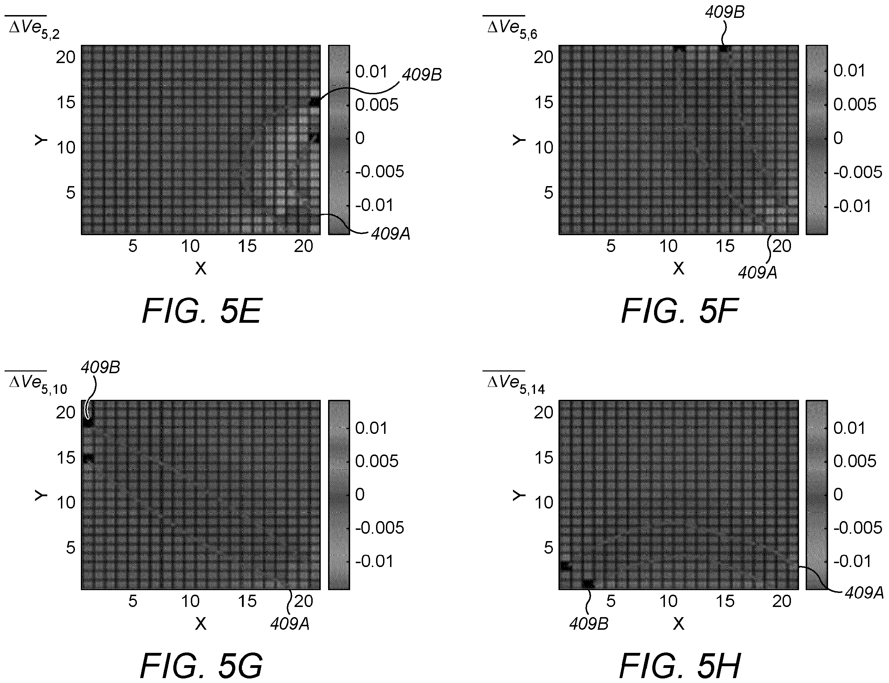

In Fig. 5E the input electrodes 409A and the output electrodes 409B are positioned on the same edge of the model. Both the input electrodes 409A and the output electrodes 409B are positioned on the right-hand edge of the model. The input electrodes 409A are positioned at a lower position of the right-hand side of the model and the output electrodes 409B are positioned at an upper position of the right-hand edge of the model.

In Fig. 5F the input electrodes 409A and the output electrodes 409B are positioned on different edges of the model. The input electrodes 409A are positioned at a lower position of the right-hand side of the model and the output electrodes 409B are positioned on the upper edge of the model.

In Fig. 5G the input electrodes 409A and the output electrodes 409B are also positioned on different edges of the model. The input electrodes 409A are positioned on the right-hand edge of the model and the output electrodes 409B are positioned on the left hand of the model. The right-hand edge of the model is opposite to the left-hand edge of the model.

In Fig. 5H the input electrodes 409A are positioned in the bottom left-hand corner of the model and the output electrodes 409B are positioned in the bottom right-hand corner of the model.

The different output signals show how the different configurations of the electrodes 409 provide different signal paths through the model and so provide different output signals. When the state of the electrodes are changed, the effect of this change can be determined for each of the pixels or voxels within the model.

This example therefore shows how the spacing and arrangement of the electrodes 409 and their activation pattern affects the output signals and the visualization of the biological sample. In Figs 5A to 5H the model is shown in two dimensions. It is to be appreciated that a three-dimensional model could be used in other implementations of the disclosure.

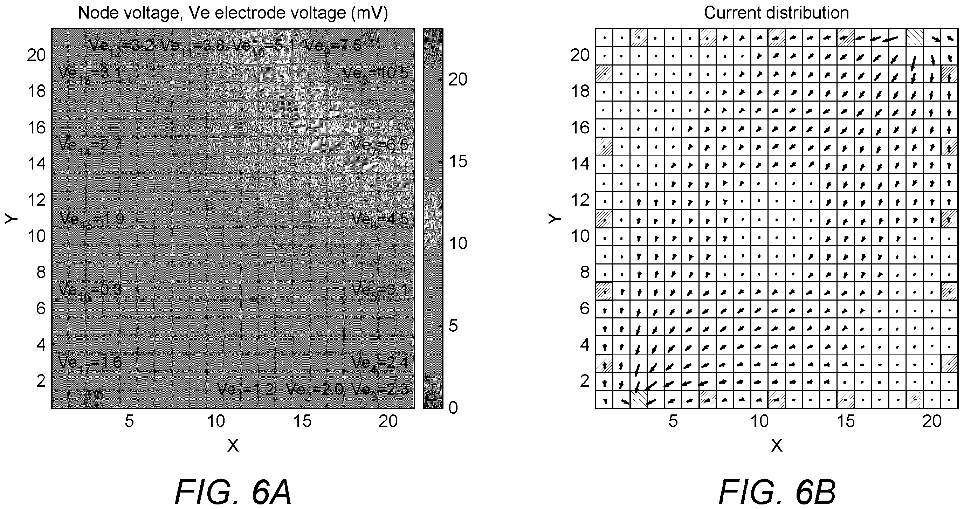

Figs. 6A and 6B show more estimated predicted output signals that can be obtained using models according to examples of the disclosure. Fig. 6A shows the output voltage distribution that is specific to the construct and layout of the biological sample and Fig. 6B shows a visualisation of the input current distribution interacting with the biological model. The visualisation of the voltage and current distribution can be obtained by running simulations using the model with different combinations of the electrode settings.

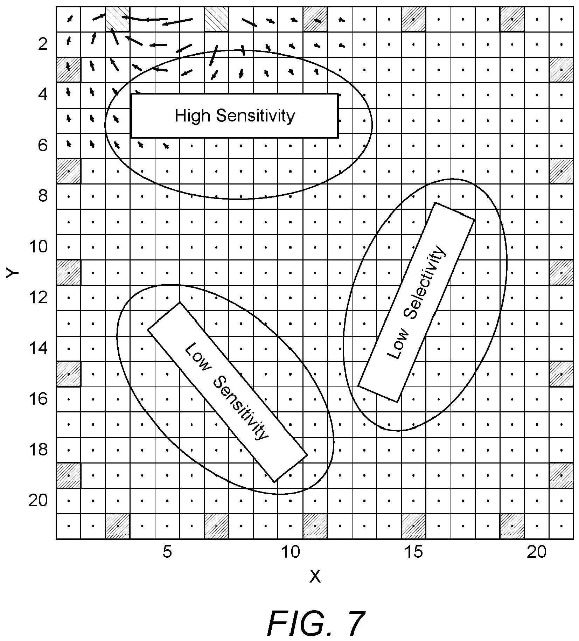

Fig. 7 shows an example visualization of estimated output signals for a two-dimensional model. This visualization can enable a user of the disclosure to identify the regions of the model, and correspondingly the regions of the biological sample, that have the highest sensitivity. This can then enable the user to configure the electrode settings on the real biological sample accordingly to determine and set various degrees of sensitivity for different regions of the biological sample.

The visualization shown in Fig. 7 is obtained by calculating and correlating different circuit-level mathematical formula in each pixel or voxel within the model for different permutations of input and output signals. This shows how uniquely differences or changes within the model, and the corresponding biological sample, can be detected. This provides a user with information indicating how sensitive or specific a region of the model, and the corresponding biological sample, are when an input current is provided and an output voltage is measured. A user can use this visualization to adjust the electrode settings on the real biological model to gain higher sensitivity in specific regions of interest. This provides higher detection accuracy of the sensor.

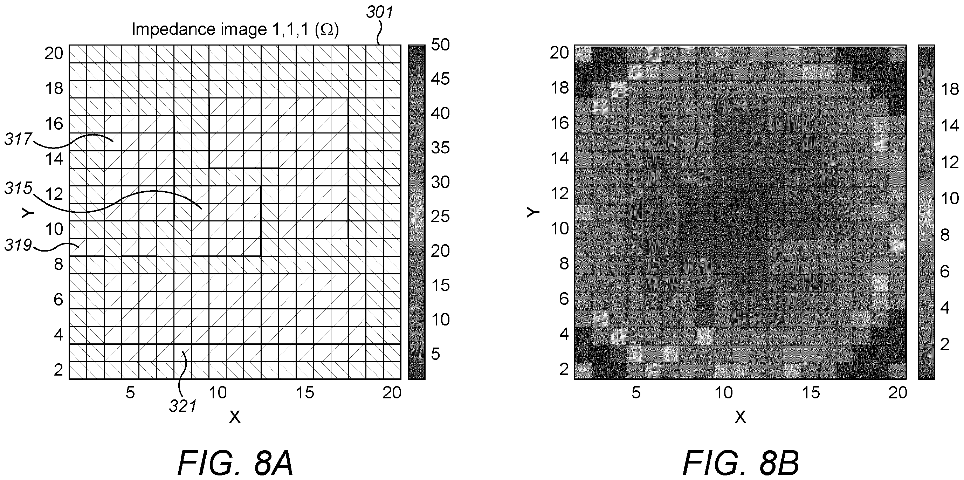

Figs. 8A and 8B shows experimental results that have been obtained using examples of the disclosure.

Fig. 8A is a two-dimensional model 301of the upper arm of a subject. This model 301 could be obtained as described above in relation to Fig. 3. A first group of squares 315 represents bone, a second group of squares 317 represent muscles, a third group of squares 319 represent arteries and a fourth group of squares represent fat 311.

Fig. 8B is a visualization that shows the different levels of sensitivity within the model 301 shown in Fig. 8A as highlighted by different shades of intensity. This intensity map shows that the regions of the lowest sensitivity are in the centre region of the model 301 which corresponds to the position of the bone. This means that for the electrode settings used to obtain these results, the centre section of the model and biological sample, will have the lowest detection accuracy.

To improve the accuracy of the results that can be obtained a user can change one or more settings of the electrodes such as electrode configuration, input or output signal patterns, information flow processing strategies or any other parameter of the sensing system. For example, the input signal delivery pattern can be configured to provide electrodes in the targeted regions of the biological sample to achieve higher sensitivity.

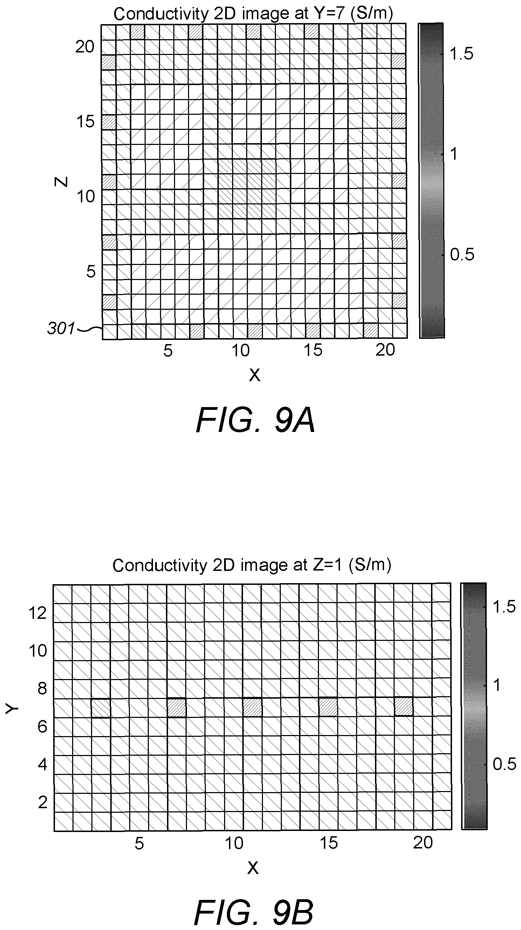

Figs. 9A to 9D show more experimental results that have been obtained using examples of the disclosure. The results have been obtained from a cross-section model of a subject's upper arm.

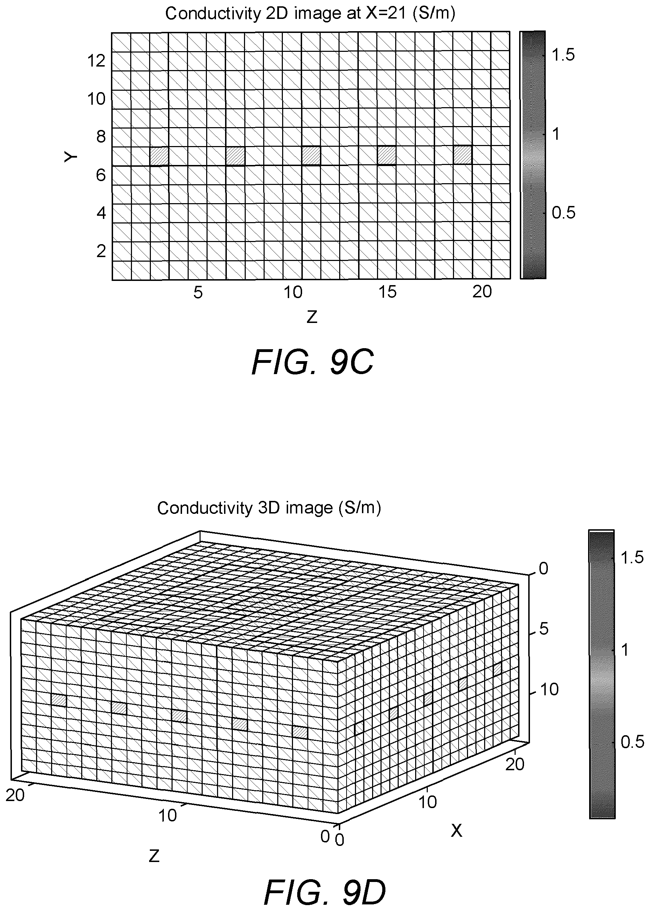

Fig. 9A shows a form of the model output in a two-dimensional conductivity image format. This highlights imprinted structures within the biological sample. This image format has input signal initiation coordinates in two-dimensions. Figs. 9B and 9C show the transformation of a two-dimensional conductivity image to a three-dimensional form with added dimensional axis, uniquely done by the system's automatic information propagation function. Fig. 9D shows the resultant three-dimensional conductivity image. This shows how two-dimensional models can be built upon to form three dimensional models with existing biological model properties propagated appropriately for 3 dimensionally analysis.

Fig. 10 schematically illustrates a controller apparatus 1001 according to examples of the disclosure. The controller apparatus 1001 illustrated in Fig. 10 can be a chip or a chip-set. In some examples the controller apparatus 1001 can be provided within a computer or other devices that be configured to provide and receive signals.

In the example of Fig. 10 the controller apparatus 1001 comprises a controller 1003. In the example of Fig. 10 the implementation of the controller 1003 can be as controller circuitry. In some examples the controller 1003 can be implemented in hardware alone, have certain aspects in software including firmware alone or can be a combination of hardware and software (including firmware).

As illustrated in Fig. 10 the controller 1003 can be implemented using instructions that enable hardware functionality, for example, by using executable instructions of a computer program 1009 in a general-purpose or special-purpose processor 1005 that can be stored on a computer readable storage medium (disk, memory etc.) to be executed by such a processor 1005.

The processor 1005 is configured to read from and write to the memory 1007. The processor 1005 can also comprise an output interface via which data and/or commands are output by the processor 1005 and an input interface via which data and/or commands are input to the processor 1005.

The memory 1007 is configured to store a computer program 1009 comprising computer program instructions (computer program code 1011) that controls the operation of the controller apparatus 1001 when loaded into the processor 1005. The computer program instructions, of the computer program 1009, provide the logic and routines that enables the controller apparatus 1001 to perform the methods illustrated in Fig. 2 The processor 1005 by reading the memory 1007 is able to load and execute the computer program 1009.

The controller apparatus 1001 therefore comprises: at least one processor 1005; and at least one memory 1007 including computer program code 1011, the at least one memory 1007 and the computer program code 1011 configured to, with the at least one processor 1005, cause the controller apparatus 1101 at least to perform: obtaining 101 a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells; using 103 the obtained model to determine expected output signals obtained in response to an electrical signal provided between two or more electrodes positioned on the biological sample; and using 105 the expected output signals to adjust one or more settings of the electrodes.

As illustrated in Fig. 10 the computer program 1009 can arrive at the controller apparatus 1001 via any suitable delivery mechanism 1013. The delivery mechanism 1013 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 1009. The delivery mechanism can be a signal configured to reliably transfer the computer program 1009. The controller apparatus 1001 can propagate or transmit the computer program 1009 as a computer data signal. In some examples the computer program 1009 can be transmitted to the controller apparatus 1001 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 1009 comprises computer program instructions for causing a controller apparatus 1001 to perform at least the following: obtaining 101 a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells; using 103 the obtained model to determine expected output signals obtained in response to an electrical signal provided between two or more electrodes positioned on the biological sample; and using 105 the expected output signals to adjust one or more settings of the electrodes.

The computer program instructions can be comprised in a computer program 1009, a non-transitory computer readable medium, a computer program product, a machine readable medium. In some but not necessarily all examples, the computer program instructions can be distributed over more than one computer program 1009.

Although the memory 1007 is illustrated as a single component/circuitry it can be implemented as one or more separate components/circuitry some or all of which can be integrated/removable and/or can provide permanent/semi-permanent/ dynamic/cached storage.

Although the processor 1005 is illustrated as a single component/circuitry it can be implemented as one or more separate components/circuitry some or all of which can be integrated/removable. The processor 1005 can be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" can refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software can not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in Fig. 2 can represent steps in a method and/or sections of code in the computer program 1009. The illustration of a particular order to the blocks does not necessarily imply that there is a required or preferred order for the blocks and the order and arrangement of the blocks can be varied. Furthermore, it can be possible for some blocks to be omitted.

The systems, apparatus 1O01, methods and computer programs 1009 can use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines can be used for supervised learning. A Bayesian network is a directed acyclic graph that represents the conditional independence of a number of random variables.

Examples of the disclosure therefore provide for apparatus, methods and computer programs for improving sensing accuracy in analysis of biological samples such as electrical impedance tomography or other suitable analysis methods. Examples of the disclosure can use information obtained from models of the electrical properties of the biological sample to configure the electrodes to provide the higher sensing accuracy. This can enable improved accuracy to be obtained with fewer electrodes or other hardware components which can improve the portability and cost-effectiveness of the analysis system.

In some examples the outputs obtained from the analysis of the biological samples could be used to provide an interface between the biological sample and a device and/or a computer program. This could enable control of the device by the biological sample. In such examples outputs from the electrodes can be used to provide a control input to a device or a computer program. For example neuromuscular changes caused by voluntary muscle action could be tracked using the analysis systems. Examples of the disclosure help to provide a system that is sensitive enough to detect these changes. This can therefore provide a system that predicts the movement of a subjects and uses this predicted movement to control external electronic devices.

In other examples the improved analysis systems could be used for monitoring a subject for the purposes of health monitoring, fitness tracking or any other suitable purpose.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells;
using the obtained model to determine expected output signals obtained in response to an electrical signal provided between two or more electrodes positioned on the biological sample; and
using the expected output signals to adjust one or more settings of the electrodes.

2. An apparatus as claimed in claim 1 wherein different sub-circuits within the model represent different types of cellular structures.

3. An apparatus as claimed in claim 2 wherein different sub-circuits representing different types of cellular structures are connected to form a representation of a tissue or organ structure.

4. An apparatus as claimed in any preceding claim wherein the sub-circuits comprise one or more electrical components configured to have substantially equivalent electrical properties to structures within a given type of cell.

5. An apparatus as claimed in any preceding claim wherein different types of sub-circuits represent each of the different type of cells and/or different types of tissues within the biological sample.

6. An apparatus as claimed in any preceding claim wherein the distribution of different types of sub-circuits within the model correspond to the distribution of different types of cells within the biological sample.

7. An apparatus as claimed in any preceding claim wherein the means are also for adjusting the model to account for changes within the biological sample.

8. An apparatus as claimed in any preceding claim wherein the means are for using the expected output signal to determine a performance of at least one of the electrodes and using the determined performance to adjust the one or more settings of the electrodes.

9. An apparatus as claimed in any preceding claim wherein the settings that are adjusted comprise at least one of: positions of one or more electrodes, types of electrodes, selection of electrodes that are used.

10. An apparatus as claimed in any preceding claim wherein the electrodes are configured to provide one or more programmable waveforms to enable tomographic analysis of the biological sample.

11. An apparatus as claimed in any preceding claim wherein outputs from the electrodes are used to provide a control input to at least one of: a device, a computer program.

12. An apparatus as claimed in claim 11 wherein the control inputs provide for an interface between the biological sample and the device to enable control of the device by the biological sample.

13. An apparatus as claimed in any preceding claim wherein the biological sample comprises an in-vivo sample.

14. A method comprising:
obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells;
using the obtained model to determine expected output signals obtained in response to an electrical signal provided between two or more electrodes positioned on the biological sample; and
using the expected output signals to adjust one or more settings of the electrodes.

15. A computer program comprising computer program instructions that, when executed by processing circuitry, cause:
obtaining a model representing electrical properties of a biological sample where the biological sample comprises a plurality of different cell types and the model comprises a plurality of different sub-circuits where the sub-circuits represent individual structures within cells of the biological sample such that the sub-circuits have electrical properties corresponding to the electrical properties of the cells;
using the obtained model to determine expected output signals obtained in response to an electrical signal provided between two or more electrodes positioned on the biological sample; and
using the expected output signals to adjust one or more settings of the electrodes.
